# EUROPEAN PATENT APPLICATION

(11) **EP 4 582 790 A1**
(43) Date of publication of application: **09.07.2025**
(21) Application number: 22969915.2
(22) Date of filing: 29.12.2022
(51) Int. Cl.: G01N 21/17, G01N 21/85

(54) **SYSTEM FOR MEASURING THE RATIO OF CURD AND WHEY**

(71) Applicant: Vila Reverter, Daniel, 17244 Cassa de la Selva (Girona) (ES)
(72) Inventor: Vila Reverter, Daniel, 17244 Cassa de la Selva (Girona) (ES)
(74) Representative: Durán-Corretjer, S.L.P.
(86) International application number: PCT/ES2022/070844
(87) International publication number: WO 2024/141681

(57) **Abstract**

Measuring system for measuring the proportion of curds and whey in a mixture of whey and curds, which comprises a sensor system and a control system, the sensor system comprising at least one light emitting element and at least one light receiving sensor, the at least one emitting element emitting a light signal, said light signal being received by at least one receiving sensor of the measuring system, the system being configured such that the signal received by the receiving sensor of the pair of sensors is transmitted to the control system in order to determine a proportion of curds in the mixture on the basis of the value of said signal.

## Description

The present invention relates to a system for measuring the proportion of curds and whey.

The system described in the present invention is a system useful for use in the food industry, for measuring the proportion of curds and for measuring the whey in a mixture of curds and whey, being especially useful for its use in the dairy industry for cheesemaking.

Curds are necessary, for example, for making cheese. Curds are produced from milk, generally milk that has been previously pasteurized. Pasteurized milk undergoes a process of coagulation in tanks to curdle. During this process, the milk is separated into curds and whey. The whey is a semi-transparent liquid substance with a yellowish colour, while the curds are a solid substance with a snow-white colour. These two substances have different properties: the curds are solid whereas the whey is liquid. In addition, they have different characteristics, such as a different density and a different opacity.

Once the milk has been coagulated and the curds and whey have been obtained, it is usual to stir the mixture of the two so that this mixture is as homogeneous as possible. However, since the densities of the two substances are not the same, the curds tend to fall to the bottom of the tank while the whey tends to remain at the top of the tank. This poses a problem, since when emptying the tank and pumping the mixture with a pump through a pipe to a dispensing machine for subsequent dispensing, the mixture that the dispensing machine receives during pumping does not have the same proportions throughout the entire process. Normally, the mixture is pumped from the bottom of the tank, and therefore, at the start of pumping the mixture has a very high curds content whereas at the end of pumping the mixture has a very low curds content. Consequently, this means that cheese produced using curds from the beginning of the tank has different properties to cheese produced using curds from the end of the tank, differing in terms of their weight and firmness, for example.

Regulation processes for regulating the concentration of curds in the mixture that is introduced into the dispensing machine are known. These processes may be manual or semi-automatic. The manual system is based on regulation of the whey extracted from the mixture by using a valve and a filter placed at the inlet of the dispensing machine. This filter allows the whey to pass through, but not the curds. The amount of whey extracted during this manual process depends on the cheesemaker or the operator who performs this regulation. The proportion of curds in the mixture is verified by analysing the moulds used during the production of the cheese. One problem with this system is that it does not allow the proportion of the mixture used to be determined and corrected directly, and it must be corrected manually after determining the properties of the final cheese initially obtained, in addition to having the disadvantage of needing human input to carry out the regulation. The semi-automatic system is based on measuring the pressure exerted by the mixture inside the dispensing machine, the whey extraction valve being operated automatically based on the value obtained by a pressure sensor. This semi-automatic regulation has the disadvantage of not being able to accurately determine the proportion of curds in the mixture used owing to the natural variability in the density of the whey and curds that make up the mixture.

It is an aim of the present invention to provide a measuring system for measuring the proportion of curds and whey in a mixture of whey and curds, as well the application of the proportion determined to regulate the processes described above and the automation thereof.

The measuring system forming the subject of the present invention comprises a sensor system and a control system. The sensor system comprises at least one light emitting element and at least one light receiving sensor. The at least one emitting element emits a light signal, said light signal being received by at least one receiving sensor of the measuring system. The system is configured such that the signal received by the at least one receiving sensor is transmitted to the control system in order to determine a value of the proportion of curds and whey in the mixture on the basis of the value of said signal. Preferably, the signal received by the receiving sensors is transmitted to the control system via an electrical connection or via a wireless connection. Note that the curds proportion value is the inverse of the whey proportion value, and therefore the curds proportion value may be obtained from a whey proportion value, and vice versa. The curds proportion value may be obtained by measuring both the curds and the whey. Preferably, the proportion value determined by the control system is a curds proportion value. Alternatively, the value determined by the control system is a whey proportion value.

The present invention is based on the fact that, owing to the very different opacities of whey and curds, the value of the signal received by the sensor depends on the quantity of curds present in the mixture, owing to the difference in opacity and transparency between the curds and the whey that make up the mixture. The light emitting element of the measuring system of the invention emits a signal to the receiving sensor of the same pair. The receiving sensor receives an amount of light corresponding to the amount of light that has managed to pass between the pieces of curds, whereas the light that encounters pieces of curds does not reach the sensor. Therefore, the intensity of the signal received is lower the higher the proportion of curds in the mixture.

Preferably, the measuring system is a system for measuring a mixture circulating in a pipe. In these cases, the light emitting element(s) and the light receiving sensor(s) are preferably distributed evenly around the pipe. Preferably, the pipe is a pipe for supplying, distributing, extracting, transferring or recirculating the mixture between two points. This pipe may be connected between a tank and a dispensing machine, or between a buffer tank and a dispensing machine. In the context of this patent application, the term "tank" refers to any tank, vat, silo, container or element in which milk, curds and/or the mixture of whey and curds are stored, or in which any of the processes for obtaining curds are carried out, including but not limited to the processes of coagulation of milk, production of curds, cutting of curds or mixing of curds and whey.

The measuring system makes it possible to find out the value of the proportion, concentration and quantity of curds and whey passing through a pipe. Knowing this value makes it possible to facilitate possible automatic regulation of the phases of said flow, such as the extraction of whey from a mixture of curds and whey so that the product or liquid dispensed into moulds always has the same weight. Measuring this value in a return circuit or after the product has stopped in a pump makes it possible to calculate the yield of a production batch and control possible losses of whey at various points in a production plant.

Preferably, the measuring system comprises a fitting for a pipe, the fitting comprising means for attachment to said pipe and a through hole to allow the passage of the mixture of curds and whey to be measured. Preferably, the emitting elements and receiving sensors are arranged in said fitting, around said hole. This fitting allows the measuring system to be installed in facilities where the mixture flows through pipes. Preferably, the fitting comprises the control system physically attached to said fitting. Alternatively, the control system is arranged externally to the fitting, electrically and/or wirelessly connected to the sensor system. For example, the control system may be arranged in an electrical box external to the fitting.

Preferably, the at least one emitting element and the at least one receiving element are arranged facing one another, forming a pair. More preferably, the at least one emitting element emits a light signal that is partially or fully received by the receiving sensor of the same pair.

The accuracy of the measurement of the curds flowing through the pipe depends on the proximity of the curds to the sensors. Preferably, the sensors are arranged around the surface of the pipe and/or the fitting. The light signal is a beam of light, preferably directed at a receiving sensor. Typically, the light signal loses intensity as the light moves through the pipe. This is usually because one of the components, in this case the curds, has a greater opacity than the other, in this case the whey, making it difficult for light to pass through. The accuracy of the measurement of the more opaque component, i.e. the curds, is high if the curds pass close to the surface, although it decreases if they pass through the centre of the pipe. This problem is especially relevant in the case of large pipes. For this purpose, the sensor system of the measuring system preferably comprises more than one light emitting element and more than one light receiving sensor. More preferably, said emitting elements and receiving sensors are evenly distributed around the perimeter of the pipe or fitting. Having several emitters-receivers makes it possible to improve measurement accuracy. Preferably, the sensor system of the measuring system comprises five light emitting elements and five light receiving sensors, more preferably evenly distributed. Particularly preferably, the emitting elements and the receiving sensors are placed in an alternating arrangement. This alternating arrangement allows uniform distribution of emitters and receivers, in turn, around the circumference of the pipe, making it possible to take suitable measurements even when the distribution of curds is not uniform in the section in which the measurement is taken.

Preferably, the light emitters are light emitting sensors. More preferably, the light emitting sensors are LED-type sensors. These light emitters may be emitters of light of any wavelength, such as visible light, ultraviolet light or infrared light emitters. These light emitters may alternatively be emitters of a known type, such as bulbs or spotlights. Preferably, the receiving sensors are photodiodes. Alternatively, the receiving sensors are photosensitive, such as phototransistors or photoresistors.

Preferably, the receiving sensors comprise an amplifier circuit. Preferably, the amplifier circuit comprises a potentiometer. This potentiometer makes it possible for all receiving sensors to provide the same maximum value. With a high gain, a small variation owing to the voltage drop of a receiving sensor could lead to a voltage difference at the output that could cause a loss of accuracy or distortion of the values measured by the measuring system. Preferably, the receiving sensors comprise a capacitor. This capacitor acts as an interference filter. This allows the grounding of values that give rise to noise after the amplifier output. Such grounding is possible by having an output voltage of the amplifier that is greater than the input voltage.

Preferably, the control system is attached to the fitting. Alternatively, the control system is connected to the fitting via attachment, coupling, fastening or anchoring means.

Preferably, the measuring system comprises a transparent cylindrical tube, the emitting elements and the receiving sensors being distributed in said cylindrical tube, more preferably uniformly distributed along said cylindrical tube. The transparent tube facilitates the transmission of the light signal without interfering with the flow of the mixture. More preferably, said transparent tube is a food-grade glass tube.

Preferably, the fitting comprises an opaque casing, said opaque casing comprising holes on its inner surface. The emitting elements and the receiving sensors are arranged over the holes in said opaque casing. This opaque casing prevents the entry of light from outside that could interfere with the readings. The opaque casing is arranged externally to the cylindrical tube.

Preferably, the control system comprises means for converting the signal received by the receiving sensors into a curds proportion value.

In pipe measurement applications, the inventor has been able to determine that there is a quadratic relationship between the signal intensity and the proportion of curds in the mixture. Therefore, on the basis of the measurement of the sensor system, the control system can determine a proportion of curds by applying a quadratic regression between the signal value received by the control system and the proportion of curds. Preferably, the control system comprises means for applying a quadratic regression to the value of the signal sent by the at least one receiving sensor to convert said signal into a curds concentration value. Preferably, the control system comprises a programmable logic controller (PLC), said controller being programmed to apply said quadratic regression. Applying a quadratic regression to the data obtained makes it possible to obtain a relationship between the values measured by the measuring system and the percentages corresponding to the quantities of between 0 - 100% of curds in the mixture. Note that the quadratic regression is a relationship determined between the signal and concentration values, and that this regression may vary depending on the technical characteristics and dimensions of the emitters, receivers or the pipe. In any event, the regression parameters may be determined empirically in each case. Alternatively, the control system may comprise a CPU and/or means for connecting to a computer, said CPU and/or computer being programmed to apply said quadratic regression so as to obtain a curds proportion value or a whey proportion value.

Preferably, the control system comprises an input protection circuit. This protection circuit is an optional protection for the control circuit. Preferably, the protection circuit comprises a diode that makes it possible to protect the circuit against reverse polarities, preferably a Schottky diode. In other words, it makes it possible to protect the control system in the event that the circuit is connected in reverse, acting as an open circuit. These diodes are faster than conventional diodes and have a low forward voltage drop. Preferably, the protection circuit comprises a varistor. This varistor acts as an open switch in the event of a voltage below the nominal voltage of the varistor, grounding the voltage in the event of a voltage surge. More preferably, it comprises capacitors. More preferably, it comprises a diode to protect the circuit against electrostatic discharge.

It is preferable that there be as much light as possible inside the pipe. This enhances the contrast of the shadows of the pieces of curds, which facilitates the reception of the signal by the receiving sensors. In an alternative embodiment, the control system comprises an attenuation circuit. More preferably, this attenuation circuit comprises an attenuator. The attenuation circuit allows the intensity of the light signal to be attenuated. This attenuation is useful in pipes of small diameter, allowing the same type of light emitting elements to be used in different sizes of pipe, attenuating their signal depending on the size of the pipe, with the attenuation circuit being particularly preferred in pipes with a diameter of less than 80 mm, especially preferred in the case where the emitting elements are LED-type sensors. More preferably, this attenuation circuit comprises a potentiometer. This potentiometer makes it possible to regulate the brightness of the emitting sensors, that is, the intensity of the light signal emitted. Alternatively, the attenuation circuit comprises a fixed resistor instead of a potentiometer.

Preferably, the control system comprises a summing amplifier circuit. This summing amplifier circuit allows electronic summing of the values received by the receiving sensors. This amplifier circuit may be a non-inverting or inverting summing amplifier circuit, preferably a non-inverting circuit. This summing amplifier circuit makes it possible to obtain the sum of the signals from the receiving sensors electronically, obtaining a summed value, and amplify said value. This circuit makes it possible to obtain an accurate value even under turbulent measurement conditions and/or in mixtures with a low curds concentration, conditions in which it could happen that only one receiving sensor sends a curds detection signal. Preferably, the non-inverting summing amplifier circuit comprises a potentiometer. This potentiometer makes it possible to regulate the gain of the amplifier. If an inverting circuit is used, the resulting signal would have a negative value, obtaining the same result as with a non-summing circuit but with reverse polarity.

Preferably, the control system comprises a voltage to current converter. This voltage to current converter makes it possible to reduce electromagnetic interference, such as that generated by signal transmission over long distances, providing greater stability to the measuring system. This converter is preferably an integrated circuit. More preferably, the converter is arranged at the output of the non-inverting summing amplifier. Preferably, the converter comprises a potentiometer for regulating the output lower limit of the converter, a potentiometer for regulating the upper limit, and a potentiometer for regulating the resolution of the summing amplifier. Preferably, the converter is a 0 - 10 V to 4 - 20 mA converter. More preferably, the amplifier amplifies the signal from the receiving sensors to obtain a signal between 0 - N V in each one, where N is the quotient between 10 and the number of receiving sensors in the measuring system. The potentiometers allow the measuring system to be configured between the lower limit (4 mA) and the upper limit (20 mA). This regulation is useful because not all types of milk produce curds with the same characteristics, depending on the fat content of the milk, the coagulation process that has been followed and the material used to curdle the milk.

In short, the light signals emitted by the light emitting element are received by the light receiving sensors. The signal received by the receiving sensor is preferably converted into a signal with a range established between 0 - N V. This resulting signal is preferably added to the resulting signals of other receiving sensors and amplified, preferably to a value between 0 and 10 V. In the case where there are five light receiving sensors, the signals received by the receiving sensors are preferably amplified to a value between 0 and 2 V. This signal intensity value is sent to the control system. This amplified and summed signal is preferably converted into current values, preferably between 4 and 20 mA. The control system will interpret this signal and obtain a curds proportion value. This interpretation is performed by virtue of a quadratic regression obtained from the graph resulting from the proportion of curds as a function of the value of the signal received. Additionally, by having a receiving sensor placed opposite the emitting element, most of the light signal emitted by an emitting element is received by the receiving sensor of the same pair.

Preferably, the measuring system comprises a curds tank. This tank may contain pasteurized milk, curds or a mixture of curds and whey before it is circulated through a pipe. Preferably, the measuring system comprises a pipe for circulating the curds and whey mixture through said pipe preferably to a dispensing machine. Preferably, the measuring system comprises a pump for pumping the mixture through said pipe.

The present invention also provides a fitting for a pipe comprising a through hole for the passage of a mixture of curds and whey, the fitting comprising a measuring system as described above, the at least one emitting element and the at least one receiving sensor being arranged in said fitting.

The present invention also provides a method for regulating a system for extracting whey from a mixture of curds and whey, said whey extraction system comprising a valve for regulating the extraction of whey, which includes:
- arranging a measuring system according to the present invention in a pipe for conveying a mixture of curds and whey,
- measuring the proportion of curds and/or whey in the mixture circulating in the pipe using the measuring system,
- regulating the valve of the whey extraction system using the curds proportion value obtained so as to extract a given quantity of whey to obtain a desired final quantity of curds.

Preferably, the whey extraction system is an extraction system of a dispensing machine. Preferably, the extraction system comprises a filter, said valve regulating the inclination of the filter. The precipitation of whey in the filter is greater if the filter is arranged in a position closer to the horizontal that allows the precipitation of the whey by gravity.

This method allows automatic regulation to extract the whey necessary to always have the same mixture and the same weight of the cheese after dispensing.

For a clearer understanding of the present invention, drawings depicting an example embodiment of the subject of the invention are attached by way of explanatory but non-limiting example.
Figure 1 shows an elevation view of a first example embodiment of a measuring system according to the present invention.
Figure 2 shows a perspective view of the example in Figure 1.
Figure 3 shows a second elevation view of the measuring system of the example embodiment in Figure 1.
Figure 4 shows a schematic view of the transmission of the light signal between the emitting elements and the receiving sensors.
Figure 5 shows a perspective view of a second example embodiment of a measuring system according to the present invention.
Figure 6 shows a perspective view of a third example embodiment of a measuring system according to the present invention.
Figure 7 shows the electrical diagram of an emitting sensor according to an example embodiment of the invention.
Figure 8 shows the electrical diagram of a receiving sensor according to an example embodiment of the invention.
Figure 9 shows the electrical diagram of an input protection circuit according to an example embodiment of the invention.
Figure 10 shows the electrical diagram of a converter according to an example embodiment of the invention.
Figure 11 shows the electrical diagram of an attenuation circuit according to an example embodiment of the invention.
Figure 12 shows the electrical diagram of a summing amplifier circuit according to an example embodiment of the invention.
Figure 13 shows the electrical diagram of a signal converter according to an example embodiment of the invention.
Figure 14 shows the electrical diagram of an output connector according to an example embodiment of the invention.

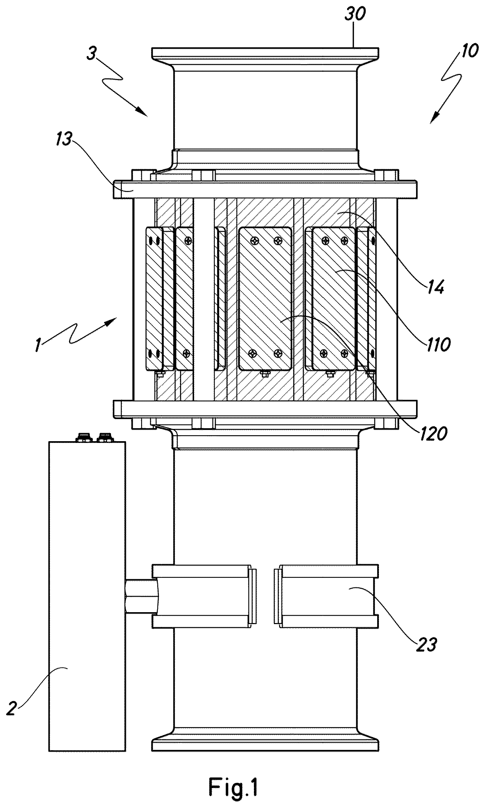

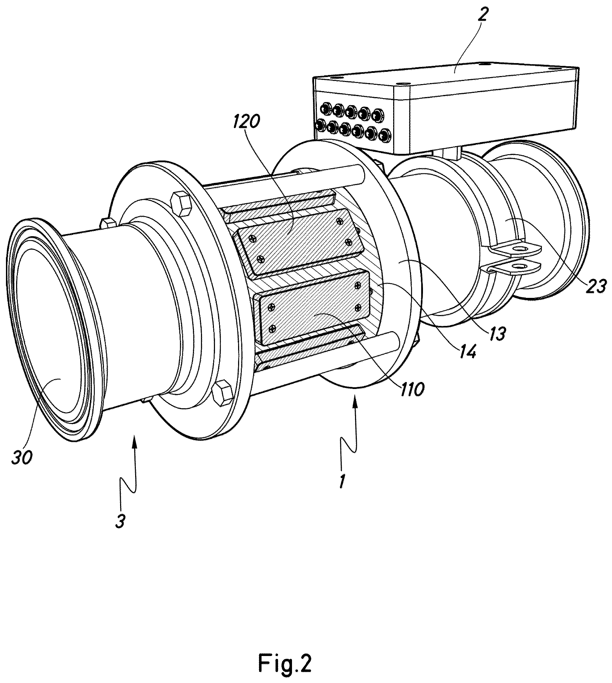

Figures 1 and 2 show a first example of a measuring system 10 according to the present invention. In the example of Figure 1, the measuring system 10 is arranged in a fitting 3 for a pipe 4. The fitting 3 comprises a tubular hole 30 for the passage of product.

The measuring system 10 comprises a sensor system 1 and a control system 2. The sensor system 1 in the example comprises emitting elements 11 which are LED-type light emitting sensors and light receiving sensors 12 which are photodiodes.

The fitting 3 comprises a cylindrical tube 13 made of transparent material and an opaque casing 14, the elements 11 and the sensors 12 being distributed in said opaque casing 14. The opaque casing 14 has been depicted using hatched lines. The opaque casing 14 comprises holes 140 that allow the transmission of the light signal from the emitting element 11 to the receiving sensor 12 through the cylindrical tube 13 made of transparent material. These holes 140 are spyholes. Each of the emitting elements 11 is shown arranged in an opaque casing 110. Likewise, each of the receiving sensors 12 is shown arranged in an opaque casing 120.

The measuring system 10 of Figure 1 comprises five emitting elements 11 and five receiving sensors 12. The emitting elements 11, depicted as sensors, and the receiving sensors 12 are arranged in pairs of sensors facing one another, and placed in an alternating arrangement such that each emitting element 11 is physically placed between two receiving sensors 12 and vice versa.

The control system 2 is attached to the fitting 3 via attachment and/or fastening means 23. In Figure 1, this attachment means 23 is a hose clamp. Alternatively, this attachment means may be a clip, a flange or an attachment and/or fastening element of known type.

In Figure 2, the opaque casing 14 has been removed so that the transparent cylindrical tube 13 can be seen.

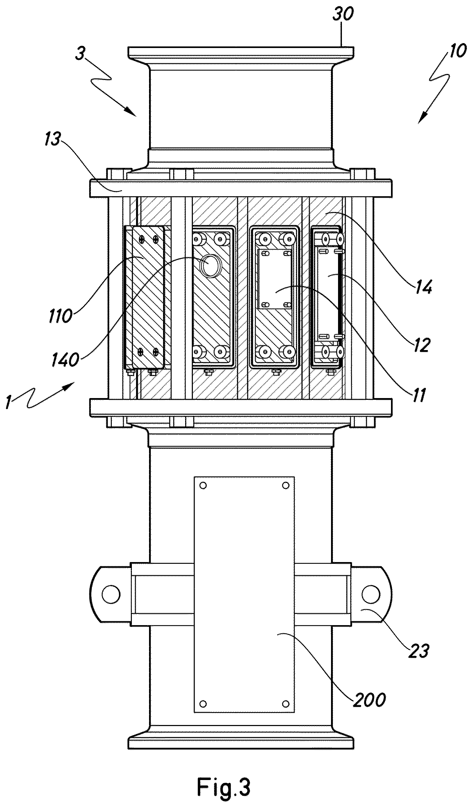

Figure 3 shows a second view of the measuring system 10 of Figures 1 and 2 in which some of the opaque casings 110, 120 of the elements 11 have been removed, along with the casing of the control system 2 for a clearer understanding of the invention. Figure 3 shows, from left to right, a receiving sensor 12 without its casing 120, an emitting sensor without its casing 110, and a hole 140 without the receiving sensor 12 or its respective casing 120 arranged thereon. As can be seen, placing the emitting elements 11 and receiving sensors 12 over the holes 140 facilitates the transmission of the light signal through the transparent cylindrical tube 13. The control system 2 has a PCB 200 plate inside it.

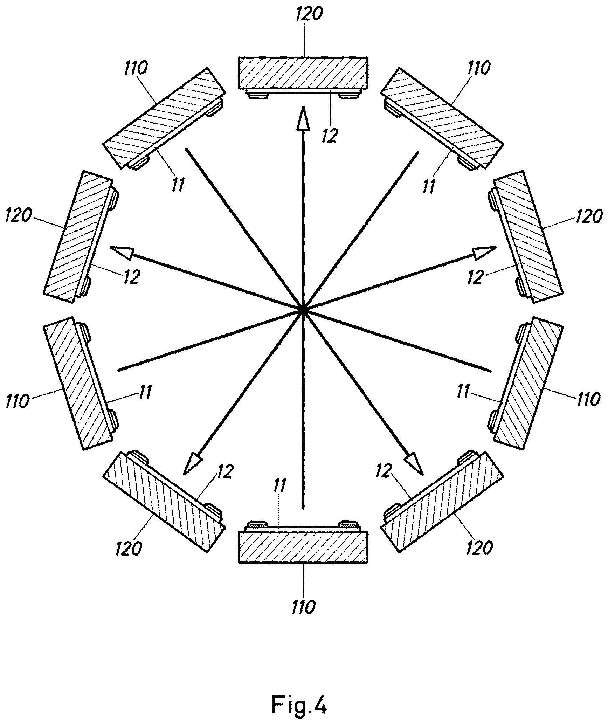

Figure 4 schematically depicts the transmission of signals from the emitting elements 11 to the receiving sensors 12. As can be seen, each emitting element 11 emits a light signal. This signal is received by the receiving sensors 12. In Figure 4, the emitting elements 11 are shown arranged with a receiving sensor 12 placed opposite it forming a pair. To facilitate understanding of the figure, the light signal received by the receiving sensor 12 has been depicted in Figure 4 as the signal coming from the emitting element 11 of the same pair even though the light signal from the emitting elements is a beam of light that is dispersed, said signal being received by more than one receiving sensor 12. The emitting elements 11 and the receiving sensors 12 are placed in an alternating arrangement, such that both the emitting elements 11 and the receiving sensors 12 are evenly distributed across the surface of the pipe, facilitating data measurement.

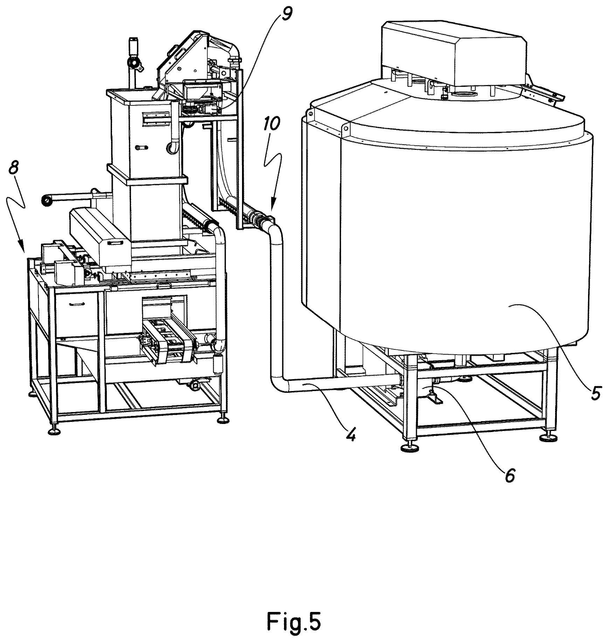

Figure 5 shows a second example of the measuring system 10. The measuring system 10 comprises a pipe 4, the measuring system 10 being attached to the pipe 4 by means of the fitting 3. The measuring system 10 also comprises a tank 5 and a pump 6 for pumping the mixture through the pipe 4. The tank 5 contains the mixture of whey and curds resulting from the coagulation of pasteurized milk. The pipe 4 is a pipe for supplying the mixture between the tank 5 and a dispensing machine 8. The dispensing machine 8 comprises a filter for extracting a phase of the mixture, for example whey. The dispensing machine 8 comprises a valve 9. This valve 9 controls the opening and/or closing of the filter. This filter may be a filter of variable size, allowing its size to be modified to extract more or less whey, depending on the curds proportion value obtained. Alternatively and/or in addition, this valve 9 makes it possible to regulate the quantity of mixture that enters the dispensing machine 8 for cheese making. The valve 9 may comprise a valve control system that can receive data on the proportion of curds in the mixture to regulate the quantity of mixture introduced into the machine 8 in order to dispense in said machine 8 a constant quantity of curds regardless of the proportion of the curds.

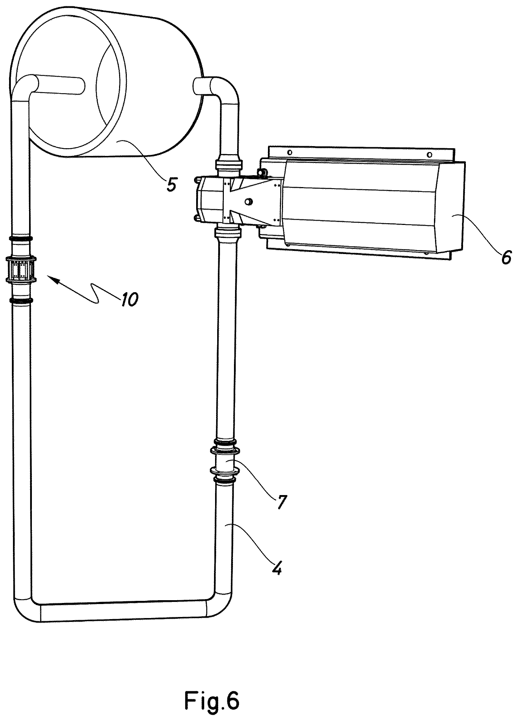

Figure 6 shows a third example of the measuring system 10, in which the pipe 4 is arranged to allow recirculation of the mixture through the pipe to the tank 5. This measuring system 10 allows the operation of the sensor system 1 and the control system 2 of the measuring system 10 to be verified. This recirculation is also useful for checking the relationship between the intensity values measured by the sensor system 1 and the proportion of the phase of the mixture to be measured, such as the proportion of curds, or alternatively the proportion of whey. Figure 6 also shows a transparent cylindrical tube 7 arranged in the pipe 4. This tube 7 makes it possible to see the mixture circulating through the pipe 4. Elements similar to those in Figure 5 have been shown with the same reference numerals.

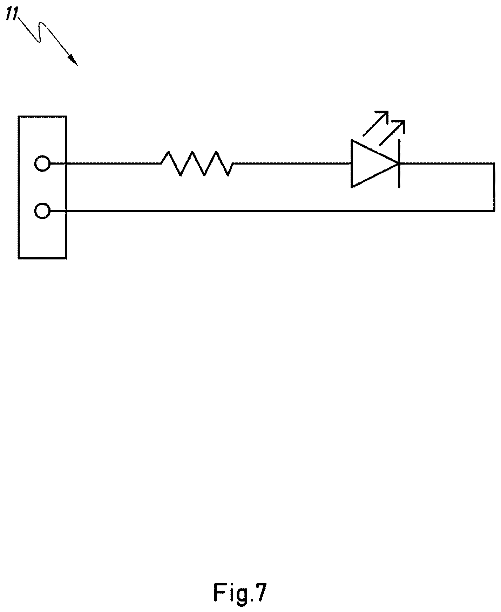

Figure 7 shows the electrical diagram 111 of an emitting element. The emitting element 11 is an LED sensor of known type.

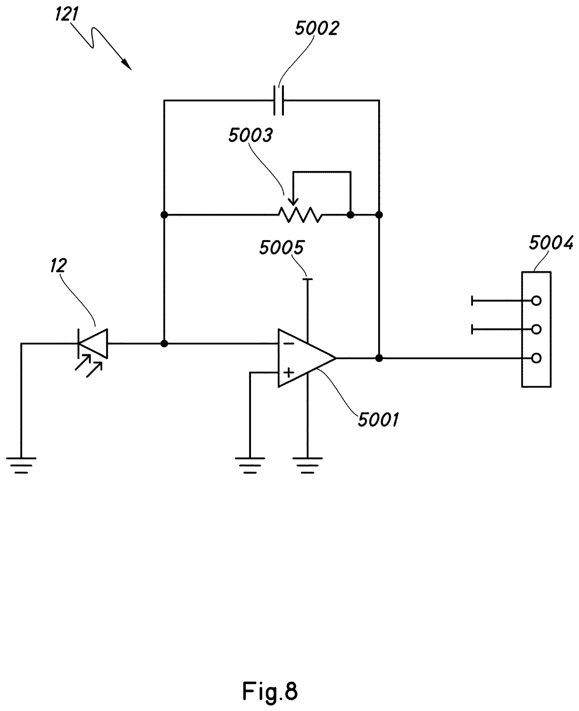

Figure 8 shows the electrical diagram 121 of a receiving sensor 12. In the example shown, the receiving sensor 12 comprises an amplification circuit that amplifies the signal received by the connector 5004. The amplification circuit comprises an amplifier 5001, as well as a potentiometer 5003 and a capacitor 5002. In this example, the potentiometer 5003 makes it possible to ensure that the receiving sensors 12 provide the same maximum voltage value. The power supply 5005 to the amplifier is a 5 V supply.

In the example embodiment shown, there are five emitting elements and five receiving sensors, the emitting elements being LED-type sensor devices and the receiving sensors being photodiodes. The photodiode amplification circuit amplifies the signal to obtain a 0 - 2 V signal on each photodiode. The non-inverting summing amplifier circuit then adds together the signals of the receiving sensors to obtain a 0 to 10 V signal. The converter then converts the 0 - 10 V signal to a 4 - 20 mA signal to reduce electromagnetic interference. In the event of a different number of receiving sensors, the amplification circuit would amplify the signal to obtain a 0 - N V signal in each photodiode, where N is the quotient between 10 and the number of photodiodes, such that the signal obtained after adding them together is between 0 and 10 V. Alternatively, other values may be used.

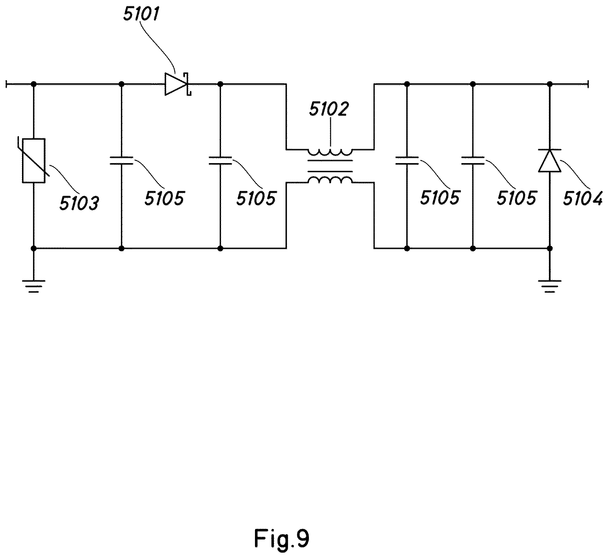

Figure 9 shows the electronic diagram of an input protection circuit of the control circuit comprising a Schottky diode 5101, an inductance 5102 and a varistor 5103, together with another diode 5104 at the output. It also comprises capacitors 5105 that act as filters. These capacitors may have the same or different materials or properties. Each of these capacitors 5105 has a different capacitance to filter different frequencies. Additionally, the control system 2 may comprise an input connector with two power lines at the input of the control system 2.

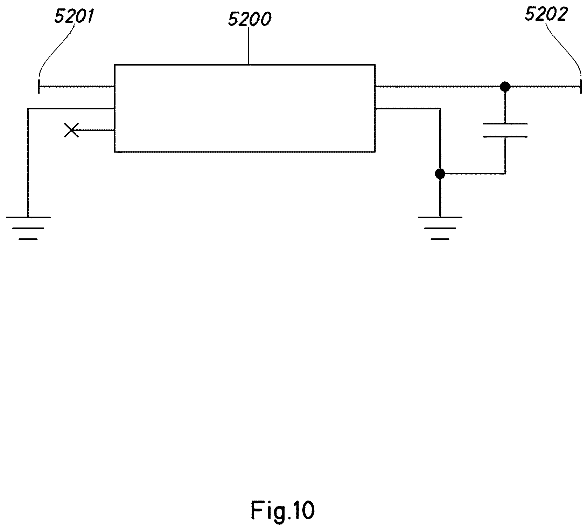

Figure 10 shows a diagram of a voltage converter. This converter 5200 allows the conversion of a 24 V input voltage 5201 to a 5 V output voltage 5202.

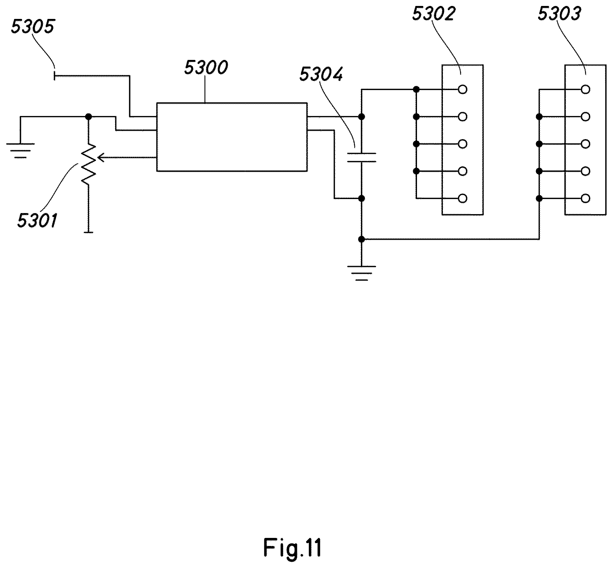

Figure 11 shows a diagram of an attenuation circuit. This attenuation circuit comprises an attenuator 5300 as well as a potentiometer 5301 for regulating the brightness of the emitting sensors. In the case shown, the connectors 5302 and 5303 are shown with five pins as there are five emitting elements and five receiving sensors in the measuring system. The attenuation circuit also comprises a filter which is a capacitor 5304. In the example shown, the attenuator input 5305 is depicted as a 24 V input.

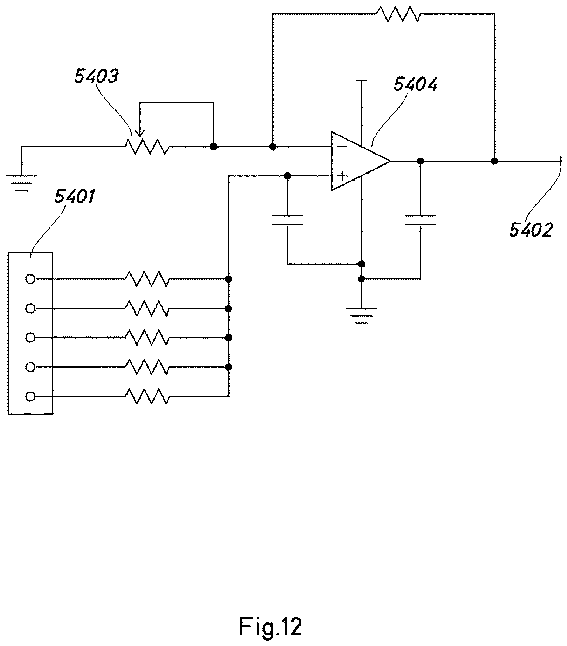

Figure 12 shows a diagram of a non-inverting summing amplifier circuit. This circuit sums the signals from the five receiving sensors depicted on a connector 5401 and generates an output signal 5402. The circuit comprises a potentiometer 5403 to regulate the gain of the amplifier 5404. In the example embodiment shown, the potentiometer 5403 regulates the gain of the amplifier 5404 to obtain an output voltage of 10 V.

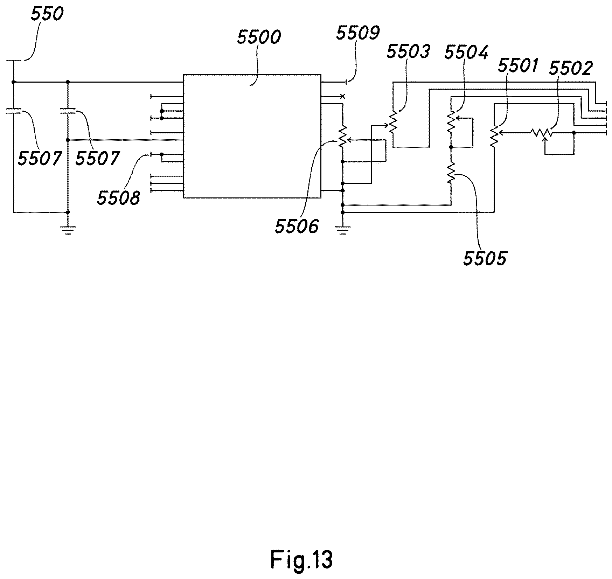

Figure 13 shows a diagram of the signal converter circuit. This circuit is shown with a potentiometer 5501 that regulates the voltage at the output of the converter 5500. In the example shown, the potentiometer 5501 is a potentiometer with a resistance of 20 kΩ. Figure 12 also shows an additional, optional potentiometer 5502 that makes it possible to increase the resolution. The circuit also shows potentiometers 5503, 5504, 5505, 5506 that regulate the signal at the converter output. In particular, the potentiometer 5503 regulates the offset of the lower limit so that it can have values other than 0 V, making it possible to shift the graph of the results obtained. Similarly, the potentiometer 5506 regulates the maximum limit, while the potentiometers 5504 and 5505 facilitate the modification of the slope of the graph resulting from the signals measured. The output 5509 of the converter 5500 and the input source 5508 of the converter are transmitted to an output connector 5600 to emit the output signal 5610, this signal being between 4 and 20 mA. The converter circuit is shown with a 24 V input 550, and with two capacitors 5507 for filtering the signal.

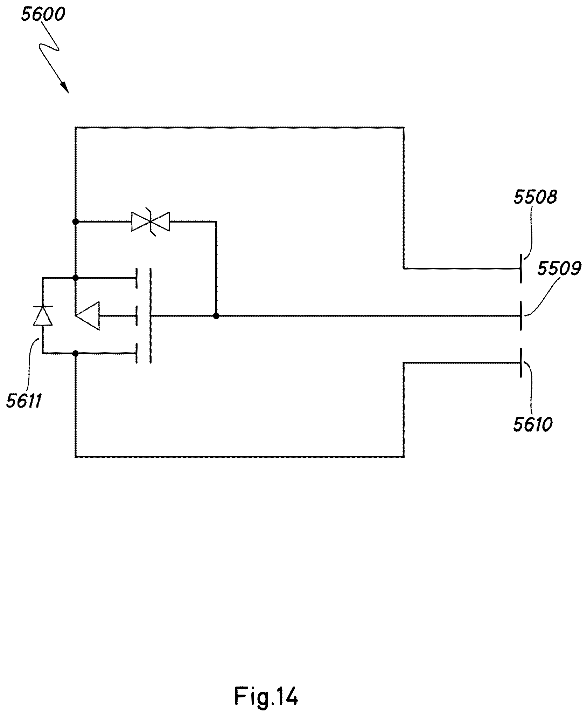

Figure 14 shows an output connector 5600 that receives the source 5508 and output 5509 signals from the converter 5500 and obtains an output signal 5610. This output connector 5600 comprises a transistor 5611. This transistor is shown as a MOSFET.

With the measured data, it is possible to configure the sensors to obtain a function that makes it possible to identify the proportion of curds circulating in a pipe. This resulting signal is not a signal directly proportional to the proportion of curds. Therefore, in the example provided by the applicant, a quadratic regression was applied to the data obtained.

Although the invention has been described with respect to preferred example embodiments, these should not be considered to be limiting on the invention, which will be defined by the broadest interpretation of the claims which follow.

## Claims

1. Measuring system for measuring the proportion of curds and whey in a mixture of whey and curds,
**characterized in that** said measuring system comprises a sensor system and a control system,
the sensor system comprising at least one light emitting element and at least one light receiving sensor, the at least one emitting element emitting a light signal, said light signal being received by at least one receiving sensor of the measuring system,
the system being configured such that the signal received by the at least one receiving sensor is transmitted to the control system in order to determine a value of the proportion of curds and whey in the mixture on the basis of the value of said signal.

2. Measuring system, according to any of the preceding claims, **characterized in that** the measuring system comprises a fitting for a pipe, the fitting comprising means for attachment to said pipe and a through hole to allow the passage of the mixture of curds and whey to be measured, the at least one light emitting element and the at least one light receiving sensor being arranged around said hole.

3. Measuring system, according to any of the preceding claims, **characterized in that** said at least one light emitting element and the at least one light receiving sensor are arranged facing one another, forming a pair.

4. Measuring system, according to Claim 3, **characterized in that** it comprises more than one light emitting element and more than one light receiving sensor, the emitting elements and the receiving sensors being placed in an alternating arrangement.

5. Measuring system, according to any of Claims 2 to 4, **characterized in that** the control system is physically attached to the fitting.

6. Measuring system, according to any of Claims 2 to 5, **characterized in that** the fitting comprises a transparent cylindrical tube, the emitting elements and the receiving sensors being uniformly distributed along said cylindrical tube.

7. Measuring system, according to any of Claims 2 to 6, **characterized in that** the fitting comprises an opaque casing, said casing comprising holes on its inner surface, the emitting elements and the receiving sensors being distributed in said casing over the holes in said opaque casing.

8. Measuring system, according to any of the preceding claims, **characterized in that** the control system comprises means for converting the signal received by the receiving sensors into a curds and whey proportion value.

9. Measuring system, according to the preceding claim, **characterized in that** the control system is programmed to apply a quadratic regression to the value of the signal sent by the at least one receiving sensor to convert said signal into a curds and whey proportion value.

10. Measuring system, according to any of Claims 4 to 9, **characterized in that** the control system comprises a summing amplifier circuit for summing the signals from the receiving sensors electronically.

11. Measuring system, according to any of the preceding claims, **characterized in that** the control system comprises a voltage to current converter.

12. Measuring system, according to Claims 10 and 11, **characterized in that** the voltage to current converter is a 0 - 10 V to 4 - 20 mA converter, and **in that** the receiving sensors are amplified to obtain a signal between 0 and N V in each one, where N is the quotient between 10 and the number of receiving sensors in the measuring system.

13. Measuring system, according to any of the preceding claims, **characterized in that** it comprises a tank for mixing curds and whey, a dispensing machine, a pipe for circulating said mixture between the tank and the dispensing machine, and a pump for pumping the mixture through said pipe.
